# EUROPEAN PATENT APPLICATION

(11) **EP 3 232 654 A1**
(43) Date of publication of application: **18.10.2017**
(21) Application number: 15867296.4
(22) Date of filing: 14.10.2015
(51) Int. Cl.: H04N 5/225, A61B 90/00, G06F 3/01, G06F 3/0484, G09G 5/00, G09G 5/36, H04N 5/228, H04N 13/00, H04N 13/02, H04N 13/04

(54) **SPECTACLE-TYPE DISPLAY DEVICE FOR MEDICAL USE, INFORMATION PROCESSING DEVICE, AND INFORMATION PROCESSING METHOD**

(30) Priority: 11.12.2014 JP 2014250721
(71) Applicant: Sony Corporation, Tokyo 108-0075 (JP)
(72) Inventor: MAEDA, Takeshi, Tokyo 108-0075 (JP); SAKAGUCHI, Tatsumi, Tokyo 108-0075 (JP)
(74) Representative: D Young & Co LLP
(86) International application number: PCT/JP2015/079044
(87) International publication number: WO 2016/092950

(57) **Abstract**

[Object] To provide a spectacle-style display device for medical use with which it is possible to change a magnification of an enlarged image, even during surgery.

[Solution] Provided is a spectacle-style display device for medical use, including: a display unit through which a surgical field is visible, and which can display an image; two imaging units that image the surgical field; an image processing unit that, on a basis of captured images imaged by the imaging units, generates a three-dimensional enlarged image to be displayed on the display unit; and a control unit that modifies a magnification of the enlarged image on a basis of a user instruction.

## Description

### Technical Field

The present disclosure relates to a spectacle-style display device for medical use, an information processing device, and an information processing method.

### Background Art

Recently, in surgical operations, there is demand to perform delicate procedures, like cardiovascular procedures, for example. When performing such surgeries, the surgeon typically uses a binocular magnifying glass such as a surgical loupe, in order to observe the surgical field stereoscopically. A binocular magnifying glass is made up of two lens barrel units, and is used by being affixed to a frame or the like worn by the user. By looking at a target object through the binocular magnifying glass, the user is able to obtain an enlarged image of the target object.

However, the magnification of the field of view by a binocular magnifying glass depends on the optical system, such as the lenses constituting the lens barrel units. Consequently, if one desires to modify the magnification of the field of view, it is necessary to replace the binocular magnifying glass. In addition, a spectacle-style monitor device provided with a zoom lens-equipped autofocus CCD camera has been proposed as a binocular magnifying glass, as illustrated in Patent Literature 1. In the spectacle-style monitor device of Patent Literature 1, the focus of the image is automatically adjusted with respect to an arbitrary set magnification, regardless of the position of the head.

### Citation List

### Patent Literature

- Patent Literature 1:: JP 3556829B

### Disclosure of Invention

### Technical Problem

However, in Patent Literature 1 above, the captured image from the CCD camera displayed on the spectacle-style monitor is displayed at a magnification set in advance, such as before surgery, and it is not possible to change the magnification during surgery.

Accordingly, the present disclosure proposes a new and improved spectacle-style display device for medical use, an information processing device, and an information processing method, with which it is possible to change the magnification of an enlarged image, even during surgery.

### Solution to Problem

According to the present disclosure, there is provided a spectacle-style display device for medical use, including: a display unit through which a surgical field is visible, and which can display an image; two imaging units that image the surgical field; an image processing unit that, on a basis of captured images imaged by the imaging units, generates a three-dimensional enlarged image to be displayed on the display unit; and a control unit that modifies a magnification of the enlarged image on a basis of a user instruction.

According to the present disclosure, there is provided an information processing device, including: an image processing unit that generates a three-dimensional enlarged image to be displayed on a display unit of a spectacle-style display device for medical use, on a basis of captured images imaged by two imaging units provided on the spectacle-style display device for medical use; and a control unit that modifies a magnification of the enlarged image on a basis of a user instruction.

According to the present disclosure, there is provided an information processing method, including: generating a three-dimensional enlarged image to be displayed on a display unit of a spectacle-style display device for medical use, on a basis of captured images imaged by two imaging units provided on the spectacle-style display device for medical use; and modifying a magnification of the enlarged image on a basis of a user instruction.

### Advantageous Effects of Invention

According to the present disclosure as described above, it is possible to change the magnification of an enlarged image, even during surgery. Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is an explanatory diagram illustrating a usage example of a spectacle-style display device for medical use according to an embodiment of the present disclosure.
[FIG. 2] FIG. 2 is a diagrammatic perspective view illustrating the appearance of a spectacle-style display device for medical use according to the embodiment.
[FIG. 3] FIG. 3 is a function block diagram illustrating a functional configuration of a spectacle-style display device according to the embodiment.
[FIG. 4] FIG. 4 is a flowchart illustrating an enlarged image display process by a spectacle-style display device according to the embodiment.
[FIG. 5] FIG. 5 is an explanatory diagram illustrating an example of a see-through display and an enlarged image superimposed display by a spectacle-style display device.
[FIG. 6] FIG. 6 is an explanatory diagram illustrating an exemplary display of an enlarged image when the wearer is regarding the left side.
[FIG. 7] FIG. 7 is an explanatory diagram illustrating an exemplary display of an enlarged image when the wearer is regarding the right side.

### Mode(s) for Carrying Out the Invention

Hereinafter, (a) preferred embodiment(s) of the present disclosure will be described in detail with reference to the appended drawings. In this specification and the appended drawings, structural elements that have substantially the same function and structure are denoted with the same reference numerals, and repeated explanation of these structural elements is omitted.

Hereinafter, the description will proceed in the following order.
1. Overview
2. Configuration of spectacle-style display device for medical use
3. Functional configuration
4. Enlarged image display process
   4.1. See-through display
   4.2. Enlarged image superimposed display
   4.3. Enlarged image hidden display

### <1. Overview>

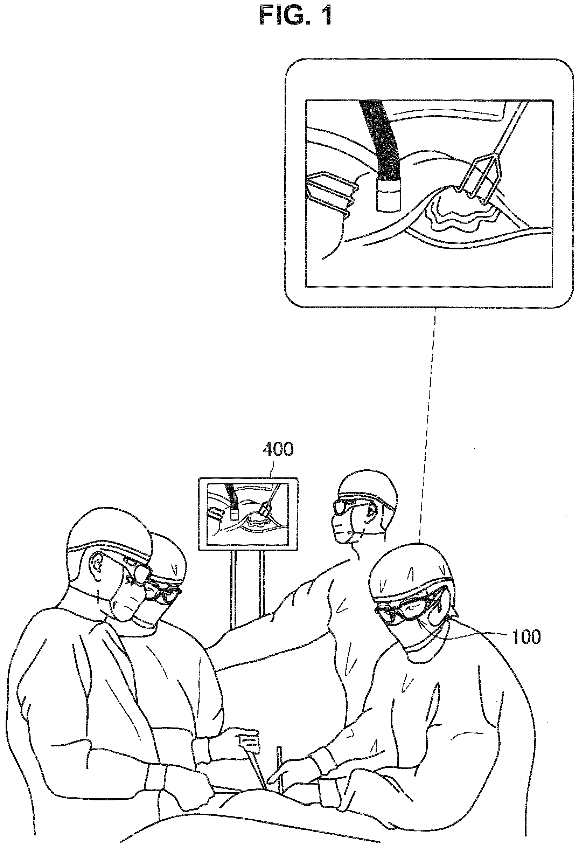

First, an overview of a spectacle-style display device for medical use according to an embodiment of the present disclosure will be described with reference to FIG. 1. FIG. 1 is an explanatory diagram illustrating a usage example of a spectacle-style display device for medical use according to the present embodiment. Note that in the following, the "spectacle-style display device for medical use" will also be designated simply the "spectacle-style display device".

The spectacle-style display device according to the present embodiment is a display device used by being worn on a user's head. The spectacle-style display device according to the present embodiment is used when enlarging and observing a surgical field with both eyes during a surgical operation, for example. The spectacle-style display device is provided with a transmissive display unit through which the outside world is transmitted and visible. The user is also able to see the outside world through the display unit, and by displaying an image on the display unit, it is possible to superimpose an image inside the field of view of the user looking at the outside world.

The spectacle-style display device 100 according to the present embodiment is used to check the details of an observation site in an abdominal surgery or the like as illustrated in FIG. 1, for example. The spectacle-style display device 100 according to the present embodiment is configured to be switchable between a see-through state, in which the observation site is shown directly through the transparent display unit, and an enlarged image superimposed state, in which an enlarged image of the observation site is displayed on the display unit. The user sets the display state to the see-through state when the user wants to obtain a field of view similar to when the spectacle-style display device 100 is not worn, such as when checking the surgical tools, looking down over the surgical field broadly, or when attempting to communicate with other staff, for example. On the other hand, the user sets and uses the spectacle-style display device 100 in the enlarged image superimposed state when the user wants to see the details of the observation site.

At this point, in the medical field, often one cannot use one's hands freely, such as during surgery. For this reason, with a binocular magnifying glass of the related art, it is also difficult to remove the binocular magnifying glass from one's field of view once worn, and the user may be required to intentionally move his or her line of site when not looking at the enlarged image. Additionally, the magnification of the binocular magnifying glass cannot be changed during surgery.

Accordingly, the spectacle-style display device 100 according to the present embodiment is configured to allow easy switching between the see-through state and the enlarged image superimposed state by a user instruction. The spectacle-style display device 100 according to the present embodiment is also configured to allow easy changing of the magnification of the enlarged image displayed in the enlarged image superimposed state by a user instruction. Hereinafter, the configuration and functions of the spectacle-style display device 100 according to the present embodiment will be described in detail.

### <2. Configuration of spectacle-style display device for medical use>

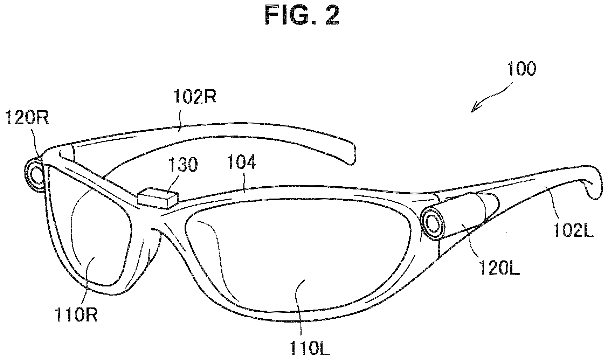

First, an exemplary configuration of the spectacle-style display device 100 according to the present embodiment will be described on the basis of FIG. 2. FIG. 2 is a diagrammatic perspective view illustrating the appearance of a spectacle-style display device for medical use according to the present embodiment.

As illustrated in FIG. 2, the spectacle-style display device 100 according to the present embodiment is made up of a frame 104 that holds lenses 110R and 110L, and supports units 102R and 102L that extend from either side of the frame 104 in a direction intersecting the lens plane. When the spectacle-style display device 100 is worn, the frame 104 is provided on front of the wearer's eyes, with the lenses 110R and 110L respectively positioned in front of the wearer's right and left eyes. The support units 102R and 102L are placed on the wearer's right and left ears, to maintain the positional relationship between the wearer's right and left eyes, and the lenses 110R and 110L. The lenses 110R and 110L of the spectacle-style display device 100 are transmissive monitors.

As illustrated in FIG. 2, the spectacle-style display device 100 according to the present embodiment is provided with two imaging units 120R and 120L that capture the outside world, such as the surgical field, and a sensor unit 103 that senses information which acts as an instruction from the user, that is, the wearer.

The imaging units 120R and 120L capture enlarged images to be displayed on the lenses 110R and 110L. In order to provide a stereoscopic display of the surgical field, the imaging units 120R and 120L are made up of an imaging unit 120R for the right eye and an imaging unit 120L for the left eye. The magnification of each of the imaging units 120R and 120L is arbitrarily modifiable by the wearer.

The imaging units 120R and 120L are provided at positions that do not obstruct the wearer's field of vision. For example, as illustrated in FIG. 2, the imaging units 120R and 120L may be provided respectively on the sides of the lenses 110R and 110L, such as at the connecting portion between the frame 104 and the support units 102R and 102L, for example. Alternatively, the imaging units 120R and 120L may be provided respectively on the top of the frame 104 so as to be positioned above the lenses 110R and 110L, or may be provided respectively on the bottom of the frame 104, so as to be positioned below the lenses 110R and 110L. The captured images imaged by the imaging units 120R and 120L are subjected to image processing by an image processing unit discussed later, and are used as enlarged images to be displayed on the lenses 110R and 110L.

The sensor unit 103 is one sensing device that senses information that acts as an instruction from the wearer. The sensor unit 103 provided on the spectacle-style display device 100 may be, for example, a speech detection device that detects the wearer's speech, or a gaze detection device that detects the wearer's gaze direction. In addition, devices such as a motion detection device that detects the motion of the wearer's head, or a gesture detection device that detects the motions of the wearer's hands and fingers as well as surgical tools handled during surgery, may also be provided as the sensor unit 103. Specifically, components such as an acceleration sensor or gyro sensor, an illuminance sensor, a microphone, and a camera may also be provided as the sensor unit 103, for example.

Note that it is sufficient to provide at least one of the sensing devices discussed above. At this point, the sensing devices may be built into the spectacle-style display device 100, or removably provided on the frame 104 or the like. Also, the switching of the display state of the spectacle-style display device 100 and the changing of the magnification of the enlarged image may also be executed on the basis of information other than the information acquired by the sensor unit 103 provided on the spectacle-style display device 100. For example, an operation input device such as a footswitch by which a user inputs information using his or her foot may also be utilized as a sensing device that senses information acting as an instruction from the wearer. The sensing devices discussed above are also not necessarily required to be provided on the spectacle-style display device 100, and as insofar as information is transmittable to an information processing unit that processes information detected by the sensing devices, the installation position of the sensing devices is arbitrary. For example, information detected by a device such as a wearable terminal being worn by the user separately from the spectacle-style display device 100 may also be utilized as information that acts as an instruction from the wearer.

In the spectacle-style display device 100, the display state of the lenses 110R and 110L is switched on the basis of information detected by sensing devices such as the sensor unit 103. Also, in the enlarged image superimposed state of the spectacle-style display device 100, the magnification of the enlarged images displayed on the lenses 110R and 110L is changed on the basis of information detected by sensing devices such as the sensor unit 103.

### <3. Functional configuration>

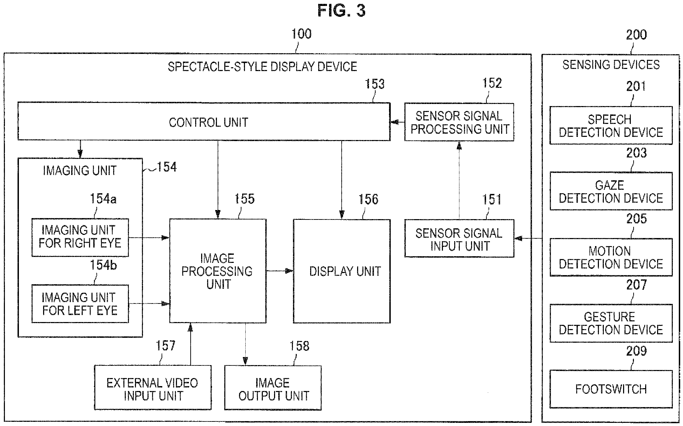

Next, a functional configuration of the spectacle-style display device 100 according to the present embodiment will be described on the basis of FIG. 3. Note that FIG. 3 is a function block diagram illustrating a functional configuration of the spectacle-style display device 100 according to the present embodiment.

As illustrated in FIG. 3, the spectacle-style display device 100 is equipped with a sensor signal input unit 151, a sensor signal processing unit 152, a control unit 153, an imaging unit 154, an image processing unit 155, a display unit 156, an external video input unit 157, and an image output unit 158.

The sensor signal input unit 151 is an interface through which signals detected by sensing devices 200 are input. As illustrated in FIG. 3, the sensing devices 200 that input signals into the sensor signal input unit 151 may be devices such as a speech detection device 201, a gaze detection device 203, a motion detection device 205, a gesture detection device 207, and a footswitch 209, for example. As discussed above, these sensing devices 200 may be provided on the spectacle-style display device 100, or may be devices separate from the spectacle-style display device 100.

For the speech detection device 201, a high-sensitivity microphone may be used, for example. For the gaze detection device 203, a camera capable of tracking the motion of the wearer's eyes, such as an infrared sensor, may be used, for example. The motion detection device 205 is a device that detects the motion of the wearer's head, for example, and a motion sensor such as an acceleration device may be used. The gesture detection device 207 is a device that detects the motions of the user's hands and fingers as well as surgical tools handled by the user, and a device such as a camera for gesture recognition may be used. The footswitch 209 is a device separate from the spectacle-style display device 100, and is used when the user inputs information using his or her foot.

Information detected by such sensing devices 200 (sensor signals) is input into the sensor signal input unit 151. The sensor signal input unit 151 outputs the input information to the sensor signal processing unit 152.

The sensor signal processing unit 152 processes information input from the sensor signal input unit 151, and generates information for causing the control unit 153 to operate suitably in response to instructions from the user, that is, the wearer. For example, the sensor signal processing unit 152 analyzes the user's spoken content from speech acquired by the speech detection device 201, and generates instruction information causing the control unit 153 to operate. The sensor signal processing unit 152 outputs the generated instruction information to the control unit 153.

The control unit 153 controls overall operation of the spectacle-style display device 100. In the display process of the display unit 156, the control unit 153 controls the operation of the imaging unit 154, the image processing unit 155, and the display unit 156 on the basis of instruction information input from the sensor signal processing unit 152.

The imaging unit 154 is an imaging unit 154 that captures an image of the outside world to obtain an enlarged image. To make the enlarged image a three-dimensional image (3D image) for stereoscopic display, the imaging unit 154 is made up of to imaging units, namely an imaging unit 154a for the right eye and an imaging unit 154b for the left eye. The magnification of the imaging unit 154a for the right eye and the imaging unit 154b for the left eye is modifiable on the basis of control information from the control unit 153. Captured images captured by the imaging unit 154a for the right eye and the imaging unit 154b for the left eye are output to the image processing unit 155. The imaging unit 154a for the right eye and the imaging unit 154b for the left eye of the imaging unit 154 correspond to the imaging units 120R and 120L in FIG. 2.

The image processing unit 155 processes captured images input from the imaging unit 154 on the basis of control information from the control unit 153, and generates an enlarged image to be displayed on the display unit 156. The image processing unit 155 performs certain image processing to adjust the parallax information of the captured images input from the imaging unit 154a for the right eye and the imaging unit 154b for the left eye and produce a stereoscopic display. The image processing unit 155 causes the display unit 156 to display the generated three-dimensional image.

The display unit 156 is a display that displays information, and in the present embodiment, is configured as a transmissive monitor. Consequently, the wearer of the spectacle-style display device 100 is able to see the outside world such as the surgical field through the display unit 156, while also being able to see an enlarged image displayed on the display unit 156. The display unit 156 corresponds to the lenses 110R and 110L in FIG. 2.

The external video input unit 157 is an interface that receives the input of video from external equipment. The external video input unit 157 outputs video input from external equipment to the image processing unit 155. Upon receiving the input of video the external video input unit 157, the image processing unit 155 conducts a process for display on the display unit 156, and causes the display unit 156 to display the video.

The image output unit 158 is an interface that outputs captured images captured by the imaging unit 154 to external equipment. The image output unit 158 outputs, to external equipment, captured images on which the image processing unit 155 has performed image processing for display on the display unit 156, for example. Consequently, an enlarged image displayed on the display unit 156 of the spectacle-style display device 100 may also be displayed on an external display 400, as illustrated in FIG. 1, for example.

The above thus describes a functional configuration of the spectacle-style display device 100 according to the present embodiment. Note that in FIG. 3, the spectacle-style display device 100 is illustrated as being equipped with the sensor signal input unit 151, the sensor signal processing unit 152, the control unit 153, the imaging unit 154, the image processing unit 155, the display unit 156, the external video input unit 157, and the image output unit 158. However, the present disclosure is not limited to such an example. For example, in the spectacle-style display device 100, components such as the sensor signal processing unit 152, the control unit 153, and the image processing unit 155 that process information for causing the imaging unit 154 and the display unit 156 to operate may also not be provided in the spectacle-style display device 100. For example, the spectacle-style display device 100 may still be made to function by causing at least some of the processes executed by these function units to be performed by an external information processing device, and transmitting the process results to the spectacle-style display device 100.

### <4. Enlarged image display process>

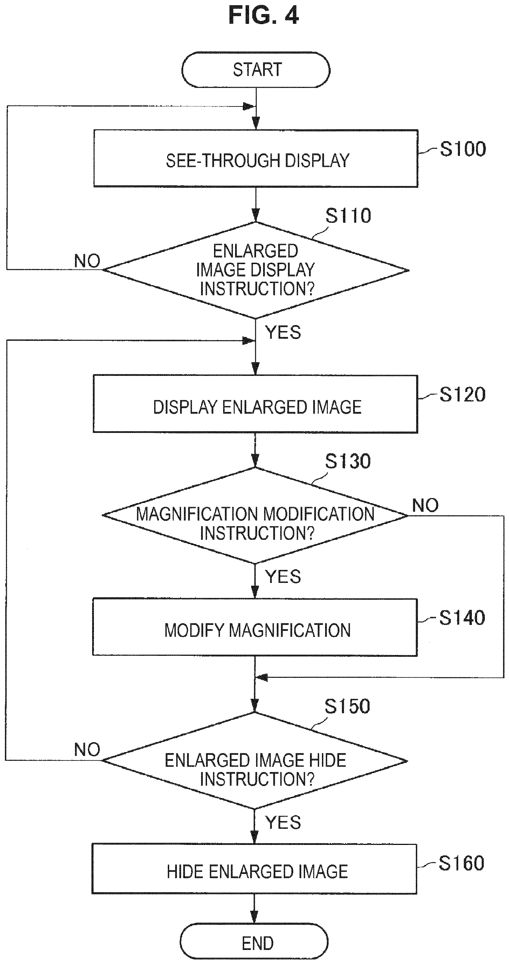

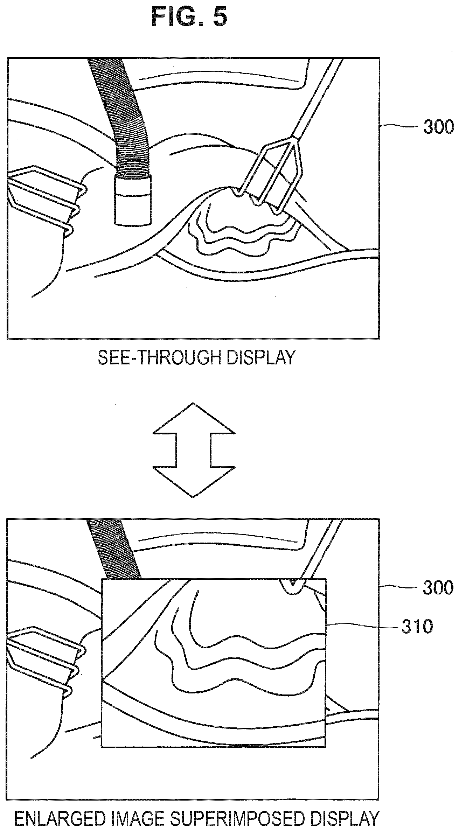

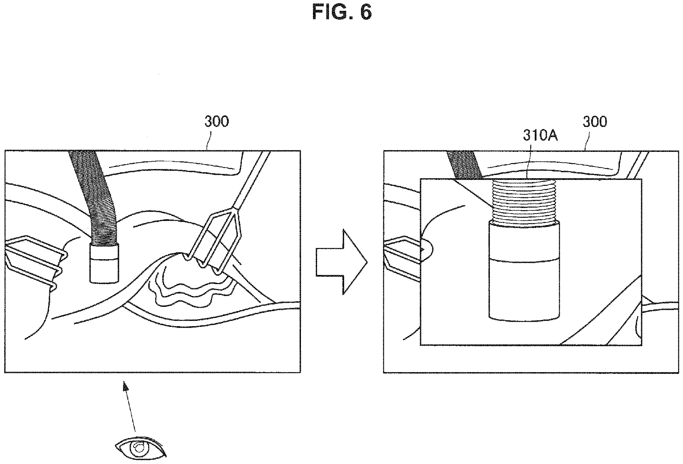

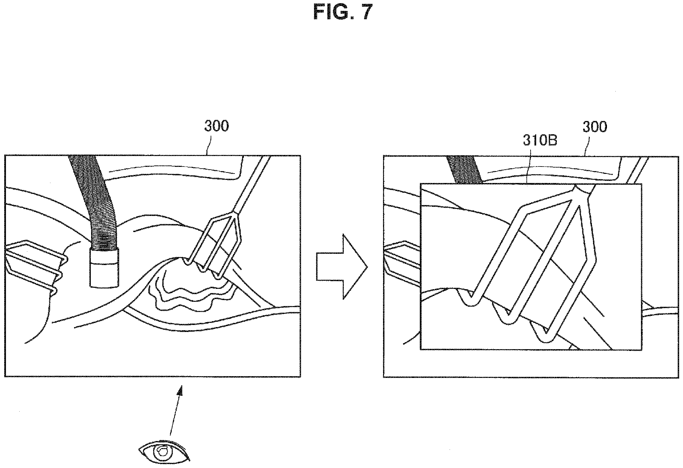

An enlarged image display process by the spectacle-style display device 100 according to the present embodiment will be described on the basis of FIGS. 4 to 7. Note that FIG. 4 is a flowchart illustrating an enlarged image display process by the spectacle-style display device 100 according to the present embodiment. FIG. 5 is an explanatory diagram illustrating an example of a see-through display and an enlarged image superimposed display by the spectacle-style display device 100. FIG. 6 is an explanatory diagram illustrating an exemplary display of an enlarged image 310A when the wearer is regarding the left side. FIG. 7 is an explanatory diagram illustrating an exemplary display of an enlarged image 310B when the wearer is regarding the right side.

### [4.1. See-through display (S100)]

As illustrated in FIG. 4, first, the display state of the spectacle-style display device 100 is set to the see-through state (S100). At this point, the wearer of the spectacle-style display device 100 is able to see the outside world directly through the display unit 156 inside a field of view 300 (the display region of the display unit 156), as illustrated in the upper part of FIG. 5, for example.

### [4.2. Enlarged image superimposed display (S110 to S150)]

### (1) Enlarged image display (S110, S120)

When the display state of the spectacle-style display device 100 is the see-through state, at a certain timing, the control unit 153 checks whether or not there is an enlarged image display instruction causing the display unit 156 to display an enlarged image from the wearer of the spectacle-style display device 100 (S110). The enlarged image display instruction is determined according to the result analyzed by the sensor signal processing unit 152 on the basis of the information detected by the sensing devices 200.

For example, if speech is acquired by the speech detection device 201, the wearer's spoken content is analyzed by the sensor signal processing unit 152. The control unit 153 determines whether or not the wearer's spoken content matches a preset enlarged image display instruction, and in the case of a match, the control unit 153 starts the process causing the display unit 156 to display an enlarged image. Information detected by other sensing devices 200 is processed similarly by the sensor signal processing unit 152 and the control unit 153, and it is determined whether or not information input by the wearer matches a preset enlarged image display instruction.

The process in step S110 is repeated until it is determined that an enlarged image display instruction has been input. Subsequently, when it is determined that an enlarged image display instruction has been input, the control unit 153 executes a process causing the display unit 156 to display an enlarged image generated from the captured images imaged by the imaging unit 154 (S120). The parallax information of the captured images is adjusted by the image processing unit 155 so that in the enlarged image, a picture similar to the field of view that the wearer can see through the display unit 156 is obtained. Subsequently, the image processing unit 155 performs certain processes to enable a stereoscopic view of the captured images, and causes the display unit 156 to display the captured images as a three-dimensional image.

At this point, the magnification of the enlarged image displayed when going from the see-through state to a state in which the enlarged image is displayed superimposed may be a preset, default magnification, or the last magnification from when an enlarged image was displayed previously. The enlarged image may be acquired by using a zoom lens-equipped camera including a CCD or CMOS image sensor as the imaging unit 154, for example, with the enlarged image set to a magnification specified by the optical zoom. As another example, the enlarged image may be generated by using an electronic zoom in which the image processing unit 155 electronically magnifies the captured images imaged by the imaging unit 154. Alternatively, a high-megapixel captured image may be acquired using a high-resolution imaging camera as the imaging unit 154, and the enlarged image may be generated by cutting out a portion of the captured image.

The enlarged image may also be generated by using the wearer's gaze direction as a reference, for example. The wearer's gaze direction is detectable from the detection results of the gaze detection device 203. Alternatively, the wearer's gaze direction may be estimated from the direction in which the imaging unit 154 is pointed, and this direction may be used as a reference to generate the enlarged image. The enlarged image may also be generated on the basis of a preset reference direction.

In addition, from the detection results of the gaze detection device 203, it is also possible to estimate a position of regard, that is, which position in the display region of the display unit 156 that the wearer is observing. This may be used to automatically switch between displaying or hiding the enlarged image, or changing the content of the enlarged image to track the wearer's position of regard.

For example, as illustrated in FIG. 6, suppose that the wearer is regarding a surgical tool on the left side of the field of view 300 of the display unit 156. At this point, if the control unit 153 recognizes the wearer's position of regard from the detection results of the gaze detection device 203, the position of regard is used as a reference to generate and display an enlarged image 310A. On the other hand, for example, as illustrated in FIG. 7, suppose that the wearer is regarding a surgical tool on the right side of the field of view 300 of the display unit 156. At this point, if the control unit 153 recognizes the wearer's position of regard from the detection results of the gaze detection device 203, the position of regard is used as a reference to generate and display an enlarged image 310B. In this way, by generating the enlarged image 310 on the basis of the wearer's position of regard, it is possible to provide an enlarged image of the site that the user wants to see.

### (2) Magnification modification (S130, S140)

After the enlarged image is displayed on the display unit 156 in step S120, at a certain timing, the control unit 153 checks whether or not there is an magnification modification instruction to modify the magnification of the enlarged image from the wearer (S130). The magnification modification instruction is determined according to the result analyzed by the sensor signal processing unit 152 on the basis of the information detected by the sensing devices 200.

For example, if speech is acquired by the speech detection device 201, the wearer's spoken content is analyzed by the sensor signal processing unit 152. The control unit 153 determines whether or not the wearer's spoken content matches a preset magnification modification instruction, and in the case of a match, the control unit 153 starts the process causing the display unit 156 to display an enlarged image at the specified magnification. As illustrated in the lower part of FIG. 5, for example, the enlarged image 310 is displayed at a certain position in the display region of the display unit 156 (for example, in the center of the display region).

Information detected by other sensing devices 200 is processed similarly by the sensor signal processing unit 152 and the control unit 153, and it is determined whether or not information input by the wearer matches a preset magnification modification instruction.

For example, when the wearer's gaze direction is detected by the gaze detection device 203, the magnification of the enlarged image may be varied in accordance with the wearer's gaze direction. For example, taking when the wearer faces forward as the reference direction, the magnification may be increased when the gaze direction is moved upward from the reference direction by a certain angle or more, and in addition, faces upward for a certain amount of time or more. On the other hand, the magnification may be decreased when the gaze direction is moved downward from the reference direction by a certain angle or more, and also faces downward for a certain amount of time or more.

Note that an enlarged image magnification modification instruction using the detection result of the gaze detection device 203 is not limited to such an example, and the magnification may also be increased when the gaze direction faces downward, and decreased when the gaze direction faces upward. Alternatively, the magnification may be modified in accordance with whether the gaze direction faces to the right side or to the left side with respect to the reference direction.

As another example, when the wearer's head motion is detected by the motion detection device 205, the magnification of the enlarged image may be varied in accordance with the wearer's head motion. The wearer's head motion may be detected using a device such as a gyro sensor, for example.

For example, taking when the wearer faces forward as a reference, the magnification may be increased when motion is detected in which the wearer faces right, and then faces forward. On the other hand, the magnification may be decreased when motion is detected in which the wearer faces left, and then faces forward. Alternatively, taking when the wearer faces forward as a reference, the magnification may be increased when motion is detected in which the wearer faces up, and then faces forward, whereas the magnification may be decreased when motion is detected in which the wearer faces down, and then faces forward. Also, the head motion that gives the instruction to modify the magnification may also be a motion of tilting the head to the left or right.

When the motions of the wearer's hands and fingers or a surgical tool handled during surgery are detected by the gesture detection device 207, the magnification of the enlarged image may be varied in accordance with these motions. These motions (gestures) may be detected by using a device such as a camera for gesture detection or an infrared camera, for example. When the control unit 153 determines that a preset magnification modification instruction gesture has been performed on the basis of an analysis result of the sensor signal processing unit 152, the control unit 153 modifies the magnification in accordance with the gesture.

For example, from the detection results of the gesture detection device 207, the magnification may be increased when it is recognized that the wearer is performing a gesture of pointing left, whereas the magnification may be decreased when it is recognized that the wearer is performing a gesture of pointing right. In this way, it becomes possible to modify the magnification of the enlarged image intuitively, in accordance with the motions of the user.

Furthermore, when operation input from the footswitch 209 is input into the sensor signal input unit 151, the control unit 153 may vary the magnification of the enlarged image in accordance with the operation input of the footswitch 209. For example, enlargement and reduction may be toggled back and forth every time the footswitch 209 is depressed, and the magnification may be varied continuously while the footswitch 209 is being depressed.

The modification of the magnification of the enlarged image may be conducted by any one of the above methods, or may be conducted by a combination of multiple methods.

In addition, the modification of the magnification of the enlarged image may be conducted in a stepwise manner or in a continuous manner. In the case of conducting the modification of the magnification of the enlarged image in a stepwise manner, multiple magnifications are preset and stored in a storage unit (not illustrated), for example. Subsequently, when there is a magnification modification instruction based on the detection results of the sensing devices 200, the magnification may be modified successively on the basis of the multiple preset magnifications.

On the other hand, in the case of modifying the magnification of the enlarged image in a continuous manner, the user indicates the start-of-modification and the end-of-modification for the magnification. When the control unit 153 recognizes that there is a magnification modification instruction on the basis of an analysis result from the sensor signal processing unit 152, the control unit 153 starts a process of continuously varying the magnification and continuously varying the displayed content of the enlarged image. The variation of the magnification is conducted until an end-of-modification instruction is recognized. Note that if a maximum configurable magnification is set, the process of modifying the magnification may end at the point in time when the maximum magnification is reached, even if there is no end-of-modification instruction.

The variation of the display content of the enlarged image may be realized by using the image processing unit 155 to electronically enlarge the captured image from the imaging unit 154, or cutting out part of a high-megapixel captured image imaged using a high-resolution camera to obtain an enlargement effect, for example.

When there is a magnification modification instruction in step S130, the control unit 153 controls the imaging unit 154, the image processing unit 155, and the display unit 156 so that the enlarged image is displayed at the newly specified magnification (S140). Note that if there is no magnification modification instruction in step S130, the flow proceeds to the process in step S150.

If the magnification is modified in step S140, the parallax experienced by the wearer changes. At this point, the image processing unit 155 may suitably adjust the generated enlarged image so that the parallax experienced by the wearer does not produce discomfort. For example, when the specified magnification is a high magnification equal to or greater than a certain magnification, excessive parallax may be produced. Accordingly, the image processing unit 155 may perform control so that the excessive parallax is not imposed on the wearer, such as by switching the enlarged image from a three-dimensional image to a two-dimensional image. A two-dimensional image may be generated by using either one of the captured images from the imaging unit 154a for the right eye or the imaging unit 154b for the left eye, for example.

### [4.3. Enlarged image hidden display (S150, S160)]

After that, the control unit 153 checks whether or not there is an enlarged image hide instruction (S150). The magnification modification instruction is determined according to the result analyzed by the sensor signal processing unit 152 on the basis of the information detected by the sensing devices 200. Likewise, the enlarged image hide instruction is determined according to the result analyzed by the sensor signal processing unit 152 on the basis of the information detected by the sensing devices 200. For example, if speech is acquired by the speech detection device 201, the wearer's spoken content is analyzed by the sensor signal processing unit 152. The control unit 153 determines whether or not the wearer's spoken content matches a preset enlarged image hide instruction, and in the case of a match, the control unit 153 starts the process causing the display unit 156 to hide the enlarged image. Information detected by other sensing devices 200 is processed similarly by the sensor signal processing unit 152 and the control unit 153, and it is determined whether or not information input by the wearer matches a preset enlarged image hide instruction.

If it is determined that an enlarged image hide instruction is input in step S150, the process from step S120 is repeated. On the other hand, when it is determined that an enlarged image hide instruction is input in step S150, the control unit 153 executes a process of hiding the enlarged image displayed on the display unit 156 (S160). After the enlarged image becomes hidden, the enlarged image display process illustrated in FIG. 4 ends.

The above thus describes an enlarged image display process by the spectacle-style display device 100 according to the present embodiment. By using the spectacle-style display device 100 according to the present embodiment, the user, that is, the surgeon is able to cause the display unit 156 to display an enlarged image and obtain an enlarged view of the surgical view easily, even in a situation in which the surgeon is holding surgical tools during surgery and is unable to use his or her hands, or in a situation in which the surgeon is unable to touch an unclean area directly. Additionally, the magnification of the enlarged image may be modified easily. Furthermore, the user is able to switch between a see-through state and an enlarged image superimposed display state, enabling safe surgery without obstructing the surgical field, even if it is necessary to check a wide-area surgical field, such as in abdominal surgery.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, in the foregoing embodiment, the enlarged image 310 displayed on the display unit 156 is taken to be a three-dimensional image, but the present technology is not limited to such an example. For example, either one of the captured images from the imaging unit 154a for the right eye or the imaging unit 154b for the left eye may be used to display a two-dimensional enlarged image, similarly to when a high magnification is set.

Also, in the foregoing embodiment, the display state of the enlarged image is switched on the basis of an instruction from the user, but the present technology is not limited to such an example. For example, in a case in which the wearer's gaze direction is detectable by the gaze detection device 203, the condition of observing the surgical field may be ascertained from the trajectory of the wearer's gaze, and the enlarged image 310 may be displayed or hidden automatically. The wearer's position of regard may be estimated from the detection results of the gaze detection device 203. Accordingly, the control unit 153 may cause the display unit 156 to display the enlarged image 310 when determining that the wearer is regarding a certain position, and hide the enlarged image 310 displayed on the display unit 156 when determining that the gaze has moved from the position of regard.

Furthermore, for an enlarged image displayed on the display unit 156 or video input externally, the display size and display orientation is not particularly limited. For example, the enlarged image or video may be displayed fullscreen in the display region 300 of the display unit 156, or may be displayed in a part of the display region 300. Also, only one enlarged image or video may be displayed in the display region 300, or multiple enlarged images or videos may be displayed in the display region 300. Likewise for the display orientation of an enlarged image or video, in the case of an enlarged image, for example, the enlarged image may be displayed in the display region 300 in an orientation similar to the images captured by the imaging unit 154, or the image may be displayed horizontally inverted, vertically inverted, or rotated in an arbitrary direction, on the basis of a user instruction or the like.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) A spectacle-style display device for medical use, including:
   a display unit through which a surgical field is visible, and which can display an image;
   two imaging units that image the surgical field;
   an image processing unit that, on a basis of captured images imaged by the imaging units, generates a three-dimensional enlarged image to be displayed on the display unit; and
   a control unit that modifies a magnification of the enlarged image on a basis of a user instruction.
(2) The spectacle-style display device for medical use according to (1), wherein
   the control unit modifies the magnification of the enlarged image on a basis of user speech.
(3) The spectacle-style display device for medical use according to (1) or (2), wherein
   the control unit modifies the magnification of the enlarged image on a basis of a user's gaze direction.
(4) The spectacle-style display device for medical use according to any one of (1) to (3), wherein
   the control unit modifies the magnification of the enlarged image on a basis of a user's head motion.
(5) The spectacle-style display device for medical use according to any one of (1) to (4), wherein
   the control unit modifies the magnification of the enlarged image on a basis of a user gesture.
(6) The spectacle-style display device for medical use according to any one of (1) to (5), wherein
   the control unit modifies the magnification of the enlarged image on a basis of an input of an operation input device operated by a user.
(7) The spectacle-style display device for medical use according to any one of (1) to (6), wherein
   the control unit modifies the magnification of the enlarged image in a stepwise manner.
(8) The spectacle-style display device for medical use according to any one of (1) to (6), wherein
   the control unit modifies the magnification of the enlarged image in a continuous manner.
(9) The spectacle-style display device for medical use according to any one of (1) to (8), wherein
   the image processing unit cuts out a portion of a captured image to generate the enlarged image.
(10) The spectacle-style display device for medical use according to any one of (1) to (9), wherein
   the image processing unit generates the enlarged image using a user's gaze direction as a reference.
(11) The spectacle-style display device for medical use according to any one of (1) to (10), wherein
   when the magnification of the enlarged image is a certain magnification or greater, the image processing unit switches the enlarged image from a three-dimensional image to a two-dimensional image for display.
(12) The spectacle-style display device for medical use according to any one of (1) to (11), wherein
   the control unit switches between displaying and hiding the enlarged image, on a basis of a user instruction.
(13) The spectacle-style display device for medical use according to any one of (1) to (12), wherein
   when the enlarged image is hidden, and the control unit determines that a user is regarding a display region from a user gaze detection result detected by a gaze detection device, the control unit cause the display unit to display the enlarged image.
(14) The spectacle-style display device for medical use according to (13), wherein
   when the control unit determines that the user's gaze has moved from a position of regard in the display region from a user gaze detection result detected by the gaze detection device, the control unit hides the enlarged image displayed on the display unit.
(15) The spectacle-style display device for medical use according to any one of (1) to (14), wherein
   the imaging units are provided at positions that do not obstruct a user's field of view from the display unit.
(16) An information processing device, including:
   an image processing unit that generates a three-dimensional enlarged image to be displayed on a display unit of a spectacle-style display device for medical use, on a basis of captured images imaged by two imaging units provided on the spectacle-style display device for medical use; and
   a control unit that modifies a magnification of the enlarged image on a basis of a user instruction.
(17) An information processing method, including:
   generating a three-dimensional enlarged image to be displayed on a display unit of a spectacle-style display device for medical use, on a basis of captured images imaged by two imaging units provided on the spectacle-style display device for medical use; and
   modifying a magnification of the enlarged image on a basis of a user instruction.

### Reference Signs List

100 spectacle-style display device
102R, 102L support unit
104 frame
110R, 110L lens
120R, 120L imaging unit
130 sensor unit
151 sensor signal input unit
152 sensor signal processing unit
153 control unit
154 imaging unit
155 image processing unit
156 display unit
157 external video input unit
158 image output unit
200 sensing devices
201 speech detection device
203 gaze detection device
205 motion detection device
207 gesture detection device
209 footswitch
300 field of view
310 enlarged image

## Claims

1. A spectacle-style display device for medical use, comprising:
a display unit through which a surgical field is visible, and which can display an image;
two imaging units that image the surgical field;
an image processing unit that, on a basis of captured images imaged by the imaging units, generates a three-dimensional enlarged image to be displayed on the display unit; and
a control unit that modifies a magnification of the enlarged image on a basis of a user instruction.

2. The spectacle-style display device for medical use according to claim 1, wherein
the control unit modifies the magnification of the enlarged image on a basis of user speech.

3. The spectacle-style display device for medical use according to claim 1, wherein
the control unit modifies the magnification of the enlarged image on a basis of a user's gaze direction.

4. The spectacle-style display device for medical use according to claim 1, wherein
the control unit modifies the magnification of the enlarged image on a basis of a user's head motion.

5. The spectacle-style display device for medical use according to claim 1, wherein
the control unit modifies the magnification of the enlarged image on a basis of a user gesture.

6. The spectacle-style display device for medical use according to claim 1, wherein
the control unit modifies the magnification of the enlarged image on a basis of an input of an operation input device operated by a user.

7. The spectacle-style display device for medical use according to claim 1, wherein
the control unit modifies the magnification of the enlarged image in a stepwise manner.

8. The spectacle-style display device for medical use according to claim 1, wherein
the control unit modifies the magnification of the enlarged image in a continuous manner.

9. The spectacle-style display device for medical use according to claim 1, wherein
the image processing unit cuts out a portion of a captured image to generate the enlarged image.

10. The spectacle-style display device for medical use according to claim 1, wherein
the image processing unit generates the enlarged image using a user's gaze direction as a reference.

11. The spectacle-style display device for medical use according to claim 1, wherein
when the magnification of the enlarged image is a certain magnification or greater, the image processing unit switches the enlarged image from a three-dimensional image to a two-dimensional image for display.

12. The spectacle-style display device for medical use according to claim 1, wherein
the control unit switches between displaying and hiding the enlarged image, on a basis of a user instruction.

13. The spectacle-style display device for medical use according to claim 1, wherein
when the enlarged image is hidden, and the control unit determines that a user is regarding a display region from a user gaze detection result detected by a gaze detection device, the control unit cause the display unit to display the enlarged image.

14. The spectacle-style display device for medical use according to claim 13, wherein
when the control unit determines that the user's gaze has moved from a position of regard in the display region from a user gaze detection result detected by the gaze detection device, the control unit hides the enlarged image displayed on the display unit.

15. The spectacle-style display device for medical use according to claim 1, wherein
the imaging units are provided at positions that do not obstruct a user's field of view from the display unit.

16. An information processing device, comprising:
an image processing unit that generates a three-dimensional enlarged image to be displayed on a display unit of a spectacle-style display device for medical use, on a basis of captured images imaged by two imaging units provided on the spectacle-style display device for medical use; and
a control unit that modifies a magnification of the enlarged image on a basis of a user instruction.

17. An information processing method, comprising:
generating a three-dimensional enlarged image to be displayed on a display unit of a spectacle-style display device for medical use, on a basis of captured images imaged by two imaging units provided on the spectacle-style display device for medical use; and
modifying a magnification of the enlarged image on a basis of a user instruction.
